# EUROPEAN PATENT SPECIFICATION

(11) **EP 2 496 274 B1**
(45) Date of publication and mention of the grant of the patent: **28.10.2015**
(21) Application number: 09749210.2
(22) Date of filing: 03.11.2009
(51) Int. Cl.: A61L 15/46, A61L 15/44, A61L 27/54, A61K 36/38, A61K 36/58

(54) **SUBSTRATE OF POLYMERIC MATERIAL AND METHOD OF CARRYING OUT THEREOF**
SUBSTRAT AUS POLYMERMATERIAL UND VERFAHREN ZUR AUSFÜHRUNG DES VERFAHRENS
SUBSTRAT DE MATÉRIAU POLYMÈRE ET PROCÉDÉ DE RÉALISATION ASSOCIÉ

(43) Date of publication of application: 12.09.2012
(73) Proprietor: Moses S.r.l., 47891 Falciano (SM)
(72) Inventor: GUALANDI, Chiara, I-40062 Molinella (Bologna) (IT); FOCARETE, Maria Letizia, I-40131 Bologna (IT); ZUCCHELLI, Andrea, I-40014 Crevalcore (Bologna) (IT); IABICHELLA, Maria Letizia, I-56127 Pisa (IT)
(74) Representative: Agazzani, Giampaolo
(86) International application number: PCT/SM2009/000009
(87) International publication number: WO 2011/056154

(56) References cited:
- US-A- 4 877 781
- US-A1- 2003 082 116
- US-A1- 2008 305 179
- US-B1- 6 579 543
- B.E. van Wyk and M. Wink: "Medicinal Plants of the World" 1 January 2004 (2004-01-01), Timber Press, Inc. , Portland (Oregon, USA) , XP002592027 ISBN: 0-88192-602-7 page 64 page 175 page 401 page 413
- QUAVE ET AL: "Dermatological remedies in the traditional pharmacopoeia ofVulture-Alto Bradano, inland southern Italy" JOURNAL OF ETHNOBIOLOGY AND ETHNOMEDICINE vol. 4, no. 5, 5 February 2008 (2008-02-05), XP002591624 DOI: 10.1186/1746-4269-4-5 Retrieved from the Internet: URL:http://www.ethnobiomed.com/content/pdf /1746-4269-4-5.pdf [retrieved on 2010-07-19]

## Description

The invention refers in particular to a substrate of a polymeric material and to the method of carrying out thereof.

Fibrous substates or supports, called scaffolds, are well known materials that can be used-as medical devices for tissue regeneration, where fibres are made of natural, synthetic, biocompatible and/or biodegradable polymers, as explained for instance in the document WO2007/132186. Scaffolds that are presently known and applied for wound healing/regeneration are characterized by several disadvantages since they are expensive (around 7-14 euros/cm²), they need to be stored in appropriate ambient conditions (controlled temperature and atmosphere); in addition, they must be applied in surgery room and their use needs a broad-spectrum antibiotic treatment.

US 2003/082116 discloses an adhesive composition comprising a polymerizable 1,1-disubstituted ethylene monomer, particularly α-cyanoacrylate monomer, and a dual-function stabilizer which could be a herbal extract. The adhesive composition is applied to a tissue and the monomer polymerizes to form a film.

US 6579543 discloses a composition having analgesic, anti-inflammatory, antioxidant and blood circulation promoting activities for the application to the skin containing St. John's wort extract.

US 4877781 discloses a composition for the hemorrhoidal tissue containing gallic acid having anti-inflammatory activity.

The main object of the present invention is to propose a substrate for wound care that is economically cheap and that can be stored also at room temperature in ambient atmosphere conditions.

Another aim of the invention is to propose a substrate that can be applied to patients in non-sterile room and that doesn't need a broad-spectrum antibiotic treatment.

A further aim is to propose a substrate useful for the preparation of a medicament that can be applied directly on the damaged tissue, such medicament having better therapeutic and wound healing properties with respect to presently existing commercial products.

The polymeric material substrate is an artificial support constituted by continuous polymer fibres, characterized by a diameter value lower than 1 µm.

Such- a substrate contains antimicrobial and/or helping tissue regeneration substances, selected among Hyperforin, Adhyperforin, I-3 Diapigenin, II-8 Diapigenin, Rutin, Quercetin, Hypericin, Azadirachtin α-β, Nimbin, Nimbidin, Salanin, Gallic Acid, Gedunin and their blends.

In the preferred embodiment of the invention the substrate contains Hyperforin, Azadirachtin α-β, or their blends, where the mixture of Hyperforin and Azadirachtin α-β is in the form of a mixture of extract of hypericum flowers in neem oil or hypericum oleolite mixed with neem oil. The substrate comprises a biocompatible polymeric material selected among polymers : polyglycolic acid (PGA), polylactic acid (PLA), copolymers of lactic and glycolic acid (PLGA), polycaprolactone (PCL), poly(3-hydroxyalkanoate) (PHA), aliphatic polycarbonates (e.g. poly(trimethylenecarbonate) (PTMC), biodegradable polyurethanes, blends and copolymers of the above mentioned polymers, polyvinylchloride (PVC), polyethylene (PE), polypropylene (PP), polymethylmetacrylate (PMMA), polystyrene (PS), polyethylenterephthalate (PET), polytetrafluoroethylene (PTFE), polyurethane (PU), polyamide (nylon).

The biocompatible substrate is also biodegradable or bioresorbable or bio-stable.

Further, the polymeric material is functionalized with at least one of the antimicrobial and/or helping tissue regeneration substances, in order to have one or more of such substances directly inserted into the macromolecular chain.

The polymeric material substrate is mesh-grafted.

In the invention the substrate is fabricated through electrospinning of the polymeric support, that later is mesh-grafted or skin-grafted.

Afterwards the polymeric material is impregnated with at least the oleous antimicrobial and/or helping tissue regeneration substance constituted preferably by a mixture of neem oil and hypericum oil.

The substrate prepared in such a way is subsequently applied onto the wound as tissue regeneration medicament.

Another embodiment of the invention consists in the preliminary mixing of the polymeric material to be electrospun with the oleous antimicrobial and/or helping tissue regeneration substance, on the subsequent electrospinning, in the mesh-grafting or skin-grafting of the artificial substrate and following application onto the wound.

Another method of substrate preparation consists in simultaneous electrospinning and electrospraying of polymer material and oleous antimicrobial and/or helping tissue regeneration substance.

Alternatively, the artificial polymeric substrate according to the invention is fabricated through electrospinning the polymeric material functionalized with one or more of said oleous antimicrobial and/or helping tissue regeneration substances, in order to have one or more of such substances directly inserted in the macromolecular chain.

The invention will be further illustrated hereinafter with reference to the following-example.

### Example

Scaffolds containing a poly(L-lactic acid) (PLLA) fibrous support and antimicrobial substances were fabricated by means of the electrospinning technology. Scaffolds characterized by two different amounts of antimicrobial substances were obtained: (i) *scaffold* 25 with a polymer : antimicrobial substances ratio of 75:25 wt:wt and (ii) *scaffold* 50 with a 50:50 wt:wt ratio. The obtained scaffolds were characterized in terms of their antibacterial activity by using *Stafilococco aureus, Pseudomonas aeruginosa* and *Escherichia coli* bacteria incubated at a 37 °C for 1h, 3h e 24h, as shown in the following table.

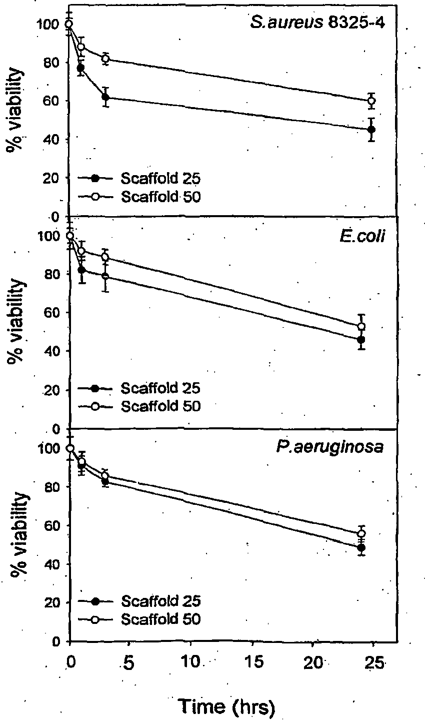

The scaffolds showed a remarkable decrease of cell viability in 24 hours (up to 60%), indicating a clear antibacterial activity. The scaffolds were mesh-grafted and directly applied on the wound.

The main advantage of the invention is to provide a cheap substrate, that can be stored also at ambient conditions and room temperature.

Another advantage is to provide a substrate that can be applied to patients in non-sterile room and that doesn't need a broad-spectrum antibiotic treatment.

A further advantage is to provide a substrate useful for the preparation of a medicament that can be applied directly on the damaged tissue, such medicament having better therapeutic and wound heating properties with respect to presently existing commercial products.

## Claims

1. Substrate composed by polymer fibres with a diameter lower than 1 µm made through the electrospinning of biocompatible and at least biodegradable or bioresorbable or bio-stable polymeric material selected among polyglycolic acid (PGA), polylactic acid (PLA), copolymers of lactic and glycolic acid (PLGA), polycaprolactone (PCL), poly(3-hydroxyalkanoate) (PHA), aliphatic polycarbonates, biodegradable polyurethanes, blends and copolymers of the above mentioned polymers,polyvinylchloride (PVC), polyethylene (PE), polypropylene (PP), polymethylmetacrylate (PMMA), polystyrene (PS), polyethylenterephthalate (PET), polytetrafluoroethylene (PTFE), polyurethane (PU), polyamide (nylon) containing at least an oleous antimicrobial and/or helping tissue regeneration substance, said substrate being **characterized in that** said at least one substance is selected among Hyperforin, Adhyperforin, I-3 Diapigenin, II-8 Diapigehin, Rutin, Quercetin, Hypericin, Azadirachtin α-β, Nimbin, Nimbidin, Salanin, Gallic Acid, Gedunin and their blends.

2. Substrate according to claim 1 **characterized in that** the mixture of Hyperforin and Azadirachtin α-β is in the form of a mixture of extract of hyperycum flowers in neem oil or hypericum oleolite mixed with neem oil.

3. Substrate according to anyone of the previous claims **characterized in that** the polymeric material is mesh-grafted.

4. Method for the preparation of a substrate according to any claims 1 to 3 **characterized by** comprising, before the electrospinning process, the blending of the polymer material to be electrospun with the at least one oleous antimicrobial and/or helping tissue regeneration substance.

5. Method for the preparation of a substrate according to any claims 1 to 3 **characterized by** comprising in addition the impregnation of the polymer material with at least one of said oleous antimicrobial and/or helping tissue regeneration substances.

6. Method for the preparation of a substrate according to any claims 1 to 3 **characterized by** comprising the electrospraying of the at least one oleous antimicrobial and/or helping tissue regeneration substance, simultaneously to the electrospinning of the polymeric material.

7. A substrate according to any of claims 1 to 3 for use as antimicrobial medicament.

8. Substrate according to any of claims 1 to 3 for use as a medicament for dermal regeneration.

## Patentansprüche

1. Substrat zusammengesetzt aus Polymerfasern mit einem Durchmesser kleiner als 1 µm, das hergestellt wurde durch das Elektrospinnen von biokompatiblem und zumindest biologisch abbaubarem, bioresorbierbarem oder biologisch stabilem polymeren Material, das ausgewählt ist aus Polyglykolsäure (PGA), Polymilchsäure (PLA), Copolymeren von Milch- und Glykolsäure (PLGA), Polycaprolacton (PCL), Poly(3-Hydroxyalkanoat) bzw. Poly(3-Hydroxylfettsäuren) (PHA), aliphatischen Polycarbonaten, biologisch abbaubaren Polyurethanen, Mischungen und Copolymeren der oben genannten Polymere, Polyvinylchlorid (PVC), Polyethylen (PE), Polypropylen (PP), Polymethylmetacrylat (PMMA), Polystyrol (PS), Polyethylenterephthalat (PET), Polytetrafluorethylen (PTFE), Polyurethan (PU), Polyamid (Nylon), das mindestens eine ölige antimikrobielle und/oder die Gewebeneubildung unterstützende Substanz umfasst, und das Substrat ist **dadurch gekennzeichnet, dass** die mindestens eine Substanz ausgewählt ist aus Hyperforin, Adhyperforin, 1-3 Diapigenin, II-8 Diapigenin, Rutin, Quercetin, Hypericin, Azadirachtin α-β, Nimbin, Nimbidin, Salanin, Gallussäure, Gedunin und seinen Mischungen.

2. Substrat gemäß Patentanspruch 1, **dadurch gekennzeichnet, dass** die Mischung aus Hyperforin und Azadirachtin α-β in Form einer Mischung aus einem Extrakt von Johanniskrautpflanzen in Neemöl oder Johanniskrautöl gemischt mit Neemöl vorliegt.

3. Substrat gemäß einem der vorhergehenden Patentansprüche, **dadurch gekennzeichnet, dass** das polymere Material in ein Gitter eingebaut wird.

4. Verfahren zur Vorbereitung eines Substrates gemäß einem der Patentansprüche 1 bis 3, **dadurch gekennzeichnet, dass** es vor dem Elektrospinnvorgang das Mischen des zu elektrospinnenden polymeren Materials mit der mindestens einen öligen antimikrobiellen und/oder die Gewebeneubildung unterstützenden Substanz aufweist.

5. Verfahren zur Vorbereitung eines Substrates gemäß einem der Patentansprüche 1 bis 3, **dadurch gekennzeichnet, dass** es zusätzlich die Imprägnierung des polymeren Materials mit mindestens einer von der öligen antimikrobiellen und/oder die Gewebeneubildung unterstützenden Substanzen umfasst.

6. Verfahren zur Vorbereitung eines Substrates gemäß einem der Patentansprüche 1 bis 3, **dadurch gekennzeichnet, dass** es das Elektrosprühen der mindestens einen öligen antimikrobiellen und/oder die Gewebeneubildung unterstützenden Substanz gleichzeitig mit dem Elektrospinnen des polymeren Materials umfasst.

7. Ein Substrat gemäß einem der Patentansprüche 1 bis 3 zur Verwendung als antimikrobielles Medikament.

8. Substrat gemäß einem der Patentansprüche 1 bis 3 zur Verwendung als Medikament für die Regenerierung der Haut.

## Revendications

1. Substrat se composant de fibres polymères ayant un diamètre inférieur à 1 µm préparées par filage électrostatique d'une substance polymère biocompatible et au moins biodégradable ou biorésorbable ou biostable choisie parmi un acide polyglycolique (PGA), un acide polylactique (PLA), les copolymères d'acide lactique et glycolique (PLGA), une polycaprolactone (PCL), un poly(3-hydroxyalcanoate) (PHA), les polycarbonates aliphatiques, les polyuréthanes biodégradables, les mélanges et les copolymères des polymères mentionnées ci-dessus, un polychlorure de vinyle (PVC), un polyéthylène (PE), un polypropylène (PP), un polyméthacrylate de méthyle (PMMA), un polystyrène (PS), un polytéréphtalate d'éthylène (PET), un polytétrafluoroéthylène (PTFE), un polyuréthane (PU), un polyamide (nylon) contenant au moins une substance oléagineuse antimicrobienne et/ou favorisant la régénération de tissus, ledit substrat étant **caractérisé en ce que** ladite au moins une substance est choisie parmi l'hyperforine, l'adhyperforine, la diapigénine I-3, la diapigénine II-8, la rutine, la quercétine, l'hypericine, l'azadirachtine α-β, la nimbine, la nimbidine, la salanine, l'acide gallique, la gédunine et leurs mélanges.

2. Substrat selon la revendication 1, **caractérisé en ce que** le mélange d'hyperforine et d'azadirachtine α-β est sous la forme d'un mélange d'extrait de fleurs d'hyperycum dans de l'huile de margousier ou d'oléolite d'hypericum mélangé à de l'huile de margousier.

3. Substrat selon l'une quelconque des revendications précédentes, **caractérisé en ce que** la substance polymère est greffée en filet.

4. Procédé de préparation d'un substrat selon l'une quelconque des revendications 1 à 3, **caractérisé en ce qu'**il comprend, avant le procédé de filage électrostatique, le mélange de la substance polymère à filer électrostatiquement avec l'au moins une substance oléagineuse antimicrobienne et/ou favorisant la régénération de tissus.

5. Procédé de préparation d'un substrat selon l'une quelconque des revendications 1 à 3, **caractérisé en ce qu'**il comprend en outre l'imprégnation de la substance polymère avec l'au moins une desdites substances oléagineuses antimicrobiennes et/ou favorisant la régénération de tissus.

6. Procédé de préparation d'un substrat selon l'une quelconque des revendications 1 à 3, **caractérisé en ce qu'**il comprend l'électropulvérisation de l'au moins une substance oléagineuse antimicrobienne et/ou favorisant la régénération de tissus, simultanément au filage électrostatique de la substance polymère.

7. Substrat selon l'une quelconque des revendications 1 à 3, pour une utilisation en tant que médicament antimicrobien.

8. Substrat selon l'une quelconque des revendications 1 à 3, pour une utilisation en tant que médicament destiné à la régénération dermique.
